# EUROPEAN PATENT APPLICATION

(11) **EP 4 520 813 A1**
(43) Date of publication of application: **12.03.2025**
(21) Application number: 24185808.3
(22) Date of filing: 01.07.2024
(51) Int. Cl.: C12M 1/00, C12M 1/34, G01N 33/18

(54) **UNDERWATER METABOLIC CHAMBER**

(30) Priority: 29.06.2023 PT 2023118770
(71) Applicant: CIIMAR - Centro Interdisciplinar de Investigação Marinha e Ambiental, 4450-208 Matosinhos (PT)
(72) Inventor: ARENAS PARRA, FRANCISCO, 4450-208 MATOSINHOS (PT); CORREIA DOS REIS, BIANCA RAQUEL, 4450-208 MATOSINHOS (PT)
(74) Representative: Patentree

(57) **Abstract**

The present disclosure relates to a portable in-situ underwater metabolic chamber for measuring metabolic parameters of an underwater sample, namely from marine communities.

## Description

### TECHNICAL FIELD

The present disclosure relates to a multisystem portable *in situ* underwater metabolic chamber for measuring metabolic parameters of an underwater sample, namely from marine communities. It relates to the following technical fields: underwater metabolic chamber, primary productivity, respiration rates, oxygen flux, *in situ* incubation, carbon cycling and climate change.

### BACKGROUND

Incubations in the laboratory are common and widespread. However usually only small individuals or pieces of the algae are used. Therefore laboratory-derived photosynthesis measurements are found to likely underestimate the productivity of complex ecosystems such as kelp forests (Rodgers et al. 2015).

Despite some progress, a major impediment has been the unavailability of an adequate chamber that can be readily attached with an effective seal, be used in multiple conditions from calm to more wave-swept, perform relative long-term incubations, during several days. To date, *in situ* primary production has been mostly measured in soft sediment ecosystems like seagrasses.

Few commercial chambers are currently available on the market. Most of them are designed for small organisms and short duration incubations. See: (CISME - https://qubitbiology.com/products/aquatic-biology/aquatic-respiration-and-photosynthesis/cisme-community-in-situ-metabolism/), or AQUATION (https://aquation.com.au/products/submersible-photosynthesis-respiration-system/).

There are other non-commercial systems that have been used for *in situ* measurements in macroalgal communities (Rodgers et al. 2015; Miller et al. 2009). The system used by Rodgers et al. (2015) focused on displacing kelp blades to different water depths and was not used for entire communities. The system by Miller et al. (2009) was developed to study foliose and turf forming macroalgal communities but is not adequately designed to harbor larger adult seaweed such as kelp.

The chambers presented in Ribeiro et al. (2003) are cages with a mesh to enclose certain species of invertebrates (mosquitos in the test described) in freshwater environments. They are designed to run toxicological tests, are partially open and do not have any sensor or flushing system.

The chambers described in the prior art presents several drawbacks, namely they do not comprise the adequate versatility to be used in different communities and performing reliable measures during several days.

Metabolic studies in underwater environments are crucial for understanding the ecological dynamics and health of aquatic ecosystems. Traditional methods of measuring metabolic parameters often involve the extraction of samples from their natural habitats, which can alter their physiological states, or inadequate chambers are used. Therefore, there is a need for a portable, in-situ device that can measure these parameters accurately without removing the samples from their environment.

These facts are disclosed in order to illustrate the technical problem addressed by the present disclosure.

### GENERAL DESCRIPTION

The present disclosure provides a reliable and multisystem portable in-situ underwater metabolic chamber, that can be used to easily obtain accurate *in situ* measurements of rates of productivity and respiration across a diverse range of conditions and marine communities without disturbing them. It is non-invasive and repeatedly deployable without requiring special technical knowledge.

The underwater metabolic chamber of the present disclosure is the only system that can be adapted to be used in different communities (benthic & pelagic) and performing reliable measures during several days. The chamber can be easily adapted to perform adult large seaweed communities. Also, the chamber described here is the only one with a flushing system.

The present disclosure provides a portable in-situ underwater metabolic chamber capable of measuring metabolic parameters of an underwater sample directly within its natural habitat. The chamber comprises a flexible incubation bag, basal frames, a sealer frame, and a flushing system to ensure sample renovation. Sensors inside the incubation bag measure various metabolic parameters, offering a comprehensive analysis of the sample's metabolic activity.

An aspect of the present disclosure relates to a portable in-situ underwater metabolic chamber for measuring metabolic parameters of an underwater sample comprising:
a flexible incubation bag (4);
a top basal frame (1) and a bottom basal frame (2);
a sealer frame (3);
a flushing system (5) for renovating the sample inside the incubation bag;
and at least a sensor inside the flexible incubation bag for measuring the metabolic parameters, preferably a plurality of sensors,
wherein:
   the flexible incubation bag comprises a closed top (7) and an open bottom (6);
   the top basal frame (1) is contiguous to the bottom basal frame (2);
   the sealer frame (3) is contiguous the bottom basal frame (2);
   the top basal frame (1), the bottom basal frame (2) and the sealer frame (3) surround the open bottom (6) of the flexible incubation bag (4).

Surprisingly, the portable in-situ underwater metabolic chamber of the present disclosure provided improved results, in particular in relation to reproducibility, tightness and precision in the measurements performed.

Regarding reproducibility, the portable in-situ underwater metabolic chamber of the present disclosure (according to example 1) has been tested in different underwater reefs from Southern to Northern areas of Portugal with improved results. Oxygen measurements that are used to calculate productivity were steady and trustworthy during light incubations, with a R² always higher than ~0.98, confirming the good functioning of the chamber (**Table 1**).

**Table 1. R² in the example incubations during light phase, revealing a steady functioning of the equipment.**

| **ID** | **R_squared** |
|---|---|
| North_1 | 0.997441 |
| North_2 | 0.997788 |
| Centre 1 | 0.995226 |
| Centre 2 | 0.985208 |
| South_1 | 0.999082 |
| South_2 | 0.998512 |

Regarding tightness, chamber's tightness was tested using a solution of fluorescein and no leaks were detected.

In an embodiment, the flushing system (5) is configured to provide a continuous flow of water through the flexible incubation bag (4).

In an embodiment, the sensor is an oxygen concentration sensor, a pH sensor and/or a temperature sensor; preferably an oxygen concentration sensor.

In an embodiment, the flushing system (5) is connected to the flexible incubation bag (4); preferably to the bottom of the flexible incubation bag (4).

In an embodiment, the top frame comprises a rubber joint.

In an embodiment, the length of the flexible incubation bag ranges from 50-200 cm; preferably 60-150 cm; more preferably 120 cm.

In an embodiment, the diameter of the flexible incubation bag ranges from 40-350 cm; preferably 40-80 cm; more preferably 50 cm.

In an embodiment, the flexible incubation bag (4) is transparent or translucid; preferably transparent.

In an embodiment, the incubation flexible bag (4) comprises a low gas permeability.

In an embodiment, the sealer frame (3) further comprises a foam joint and a surrounding plastic material (to seal the chamber to the substrate). In a preferred embodiment, the material of the foam joint of the sealer frame (3) is selected from a list consisting of: polyurethane, polyether, polyester, latex; preferably polyurethane.

In an embodiment, the material of the surrounding plastic material is selected from a list consisting of: high-density polyethylene, polyethylene terephthalate, polyvinyl chloride; preferably polyvinyl chloride.

In a preferred embodiment, the material of the flexible incubation bag (4) is selected from a list consisting of: low-density polyethylene, polypropylene; preferably polypropylene.

In an embodiment, the material of the top basal frame (1) and/or the material of the bottom basal frame (2) is selected from a list consisting of: high-density polyethylene, polyethylene terephthalate, polyvinyl chloride; preferably polyvinyl chloride.

In a preferred embodiment, the top basal frame (1) and/or the bottom basal frame (2) has a circular shape.

In an embodiment, the internal diameter of the top basal frame (1) and/or the internal diameter of the bottom basal frame (2) ranges from 40 - 80 cm; preferably 50 cm.

In an embodiment, the external diameter of the top basal frames (1) and/or the external material of the bottom basal frame (2) ranges from 60 - 100 cm; preferably 70 cm.

In a preferred embodiment, the diameter of the flexible incubation bag (4) is 50 cm; the material of the top basal frame (1) and/or the material of the bottom basal frame (2) is polyvinyl chloride and comprises a circular shape; the internal diameter of the top basal frame (1) and/or the internal diameter of the bottom basal frame (2) is 50 cm; and the external diameter of the top basal frame (1) and/or the external diameter of the bottom basal frame (2) is 70 cm.

In an embodiment, the flushing system (5) comprises:
a motorize valve;
a pump;
a power supply;
an electronic microcontroller (to activate the flush of seawater inside the chamber at intervals and intensities predefined).

In a preferred embodiment, the portable in-situ underwater metabolic chamber of the present disclosure further comprises a protective structure for the flushing system.

Another aspect of the present disclosure relates to the use of the portable in-situ underwater metabolic chamber of the present disclosure as a metabolic chamber for analyzing *in situ* a substrate in pelagic and benthic habitats.

In a preferred embodiment, the underwater metabolic chamber of the present disclosure is used as a marine metabolic chamber.

In a preferred embodiment, the underwater metabolic chamber of the present disclosure further comprises weights attached to the top basal frame (1) to immobilize and/or stabilize the underwater metabolic chamber.

In a preferred embodiment, the chamber comprises a flexible incubation bag and basal frames. Incubation bags are transparent bags, preferably with a conical shape, preferably from polypropylene, and presents low gas permeability. The length of the incubation bags ranges from 60-150 cm; preferably 120 cm; and the diameter of the incubation bag ranges from 40-80 cm; preferably approximately 50 cm but total longitude can be adapted depending on the use by folding the free extreme.

Basal frames are two solid frames, preferably PVC (polyvinyl chloride) frames with circular shape and the internal diameter ranges from 40-80 cm; preferably 50 cm and external diameter ranges from 60-100 cm; preferably 70 cm.

### BRIEF DESCRIPTION OF THE DRAWINGS

The following figures provide preferred embodiments for illustrating the disclosure and should not be seen as limiting the scope of invention.

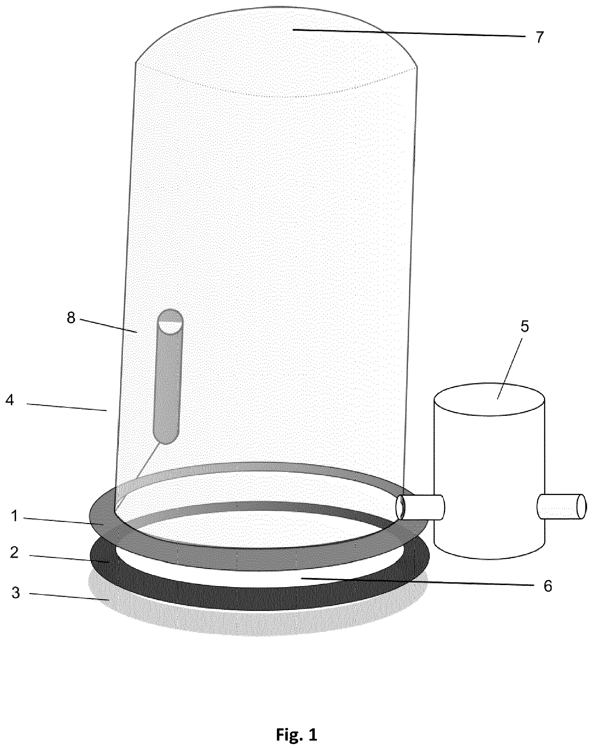

**Figure 1****:** Schematic representation of a first preferred embodiment wherein:
   (1) represents the top basal frame;
   (2) represents the bottom basal frame;
   (3) represents the sealer frame (foam joint);
   (4) represents the flexible incubation bag;
   (5) represents the flushing system;
   (6) represents the open bottom of the flexible incubation bag;
   (7) represents the closed top of the flexible incubation bag;
   (8) represents a sensor inside the flexible incubation bag.

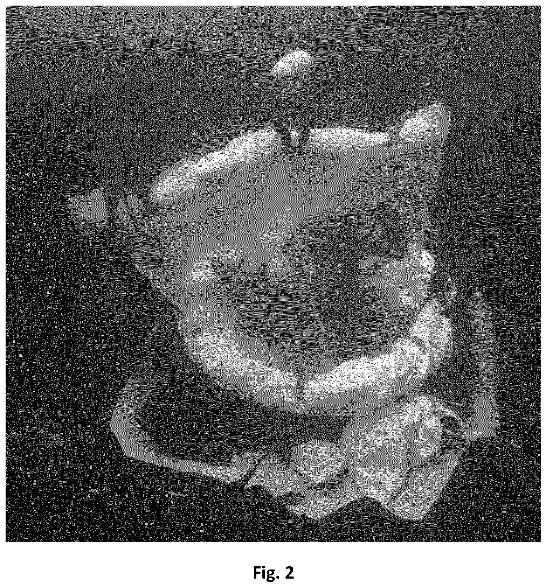

**Figure 2** represents the first prototype performing measures *in-situ.*
**Figure 3** represents the Productivity-Irradiance curve of a benthic macroalgal community in Sines, Portugal. Measurements were obtained using the underwater metabolic chamber of the present disclosure. PAR (Photosynthetic Active Radiation).

### DETAILED DESCRIPTION

The present disclosure relates to a low cost, robust and repeatedly deployable underwater metabolic chamber system that can be used to easily obtain accurate measurements of in situ rates of photosynthesis and respiration across a diverse range of conditions and marine habitats. It offers progress towards attaining an understanding of the functional performance of marine assemblages, both benthic and pelagic. The adjustable size of the incubation chamber allows the generation of metabolic rates in large seaweeds or seagrasses. The chamber has a programmable flush system to renovate seawater allowing long incubations, up to several days, allowing the estimation of daily rates. The novel photorespirometry system (underwater metabolic chamber) has large potential uses in the field of marine functional ecology and can be used in addition to other monitoring activities in order to assess changes in metabolic rates over time.

The underwater metabolic chamber of the present disclosure was designed to measure functional traits, *i.e*. metabolic rates, of whole communities in marine environments. They are deployed by divers at subtidal, where they are able to log oxygen fluxes continuously and flush regularly new seawater to prevent hypoxia in the chamber. They can also be adapted for their use in pelagic environments, where the plankton productivity rates can be estimated.

The underwater metabolic chamber of the present disclosure provides an effective seal and impermeable chamber walls suitable for rocky reef systems. Further, it provides flexibility of chamber walls to allow the natural water movements that affect the photosynthesis rates of large seaweeds such as kelps.

The chamber of the present disclosure is the only one incorporating a flush system to renovate the water when programmed, thus able to perform metabolic experiments lasting several days.

In an embodiment, the underwater metabolic chamber of the present disclosure comprises:
- A flexible incubation bag (4): The flexible incubation bag is designed to contain the underwater sample during the measurement process. It features a closed top (7) and an open bottom (6) for ease of sample insertion and containment.
- A top basal frame (1) and Bottom Basal Frame (2): These frames provide structural support for the incubation bag. The top basal frame (1) is contiguous to the bottom basal frame (2), forming a sturdy base for the chamber.
- Sealer Frame (3): The sealer frame is positioned contiguous to the bottom basal frame (2), ensuring a secure seal around the open bottom (6) of the flexible incubation bag (4). This arrangement ensures no leakage and maintains the integrity of the sample within the bag.
- Flushing System (5): The flushing system renovates the sample inside the incubation bag, preventing stagnation and ensuring a fresh supply of water to the sample. This system is essential for accurate metabolic measurements over extended periods.
- Sensors: A plurality of sensors is integrated within the flexible incubation bag to measure various metabolic parameters such as oxygen levels, carbon dioxide levels, pH, temperature, and other relevant metrics. These sensors provide real-time data and are crucial for comprehensive metabolic analysis.

The portable in-situ underwater metabolic chamber is assembled by positioning the flexible incubation bag within the top basal frame, bottom basal frame, and sealer frame. The open bottom of the bag is securely sealed by the contiguous arrangement of the frames. Once assembled, the chamber is submerged in the underwater environment where the sample is to be measured. The flushing system is activated to ensure continuous water flow through the incubation bag, and the sensors begin collecting metabolic data.

### Example 1

In the embodiment of example 1, the underwater metabolic chamber of the present disclosure comprises a flexible incubation bag, basal frames and a sealer frame. The incubation bag is a flexible, transparent polypropylene bag with low gas permeability. The polypropylene bag is 120 cm long with a diameter of 50 cm (total longitude can be adapted depending on the use by folding the free extreme). Top and bottom basal frames are two PVC frames with circular shape and internal diameter of 50 cm and external diameter of 70 cm. The basal frames are tight together with screws and hold the base of the incubation bag. To ensure water tightness between the frames, the top frame holds a rubber joint. When performing benthic incubations with the flexible bags, the chambers are sealed to the substrate preventing mixing with the surrounding water. The sealing is ensured by the sealer frame comprising a soft foam joint (polyurethane foam) and a surrounding plastic material (PVC - Polyvinyl Chloride) that is further tight to the irregular substrate by using small 2 kg sand sacs. During the incubation the flexible chamber walls provide transmission of wave energy into the chamber thus no extra pumps are required. Using jointly two chambers frames with two flexible incubator bags we can run pelagic incubations at the desired depth. The chamber comprises a programmable flush system to renovate seawater for long, up to several days, incubations allowing the estimation of daily rates. Physical conditions, temperature and oxygen concentration within the chamber are periodically monitored using commercial loggers or a purpose-built logging system. Flushing system incorporates a 12 V motorize valve, a small 12 V pump all controlled by an electronic microcontroller fed by a 12 V lithium battery that activates the flush of seawater inside the chamber at intervals and intensities programmed by the user. All these devices are included in an epoxy submersible housing.

In an embodiment, the chamber is built in a modular way to be used *in situ* on varies types of marine habitats, both pelagic and benthic habitats (including soft and rocky bottom).

In a preferred embodiment, the chamber incorporates a flush-in system to perform long duration incubations, i.e. several days.

In an embodiment, the chamber is suitable for use with commercial oxygen, temperature sensors or use its own submersible built-in sensors.

In a preferred embodiment, the chamber walls are flexible which allows seaweed/seagrasses to move naturally. The chamber size can be customized and walls can be adjusted *in situ* on the spot to height of respective community.

In an embodiment, the chamber size can be customized to size of study organisms/study area.

In an embodiment, the chamber is easily and repeatedly deployable without special technical knowledge.

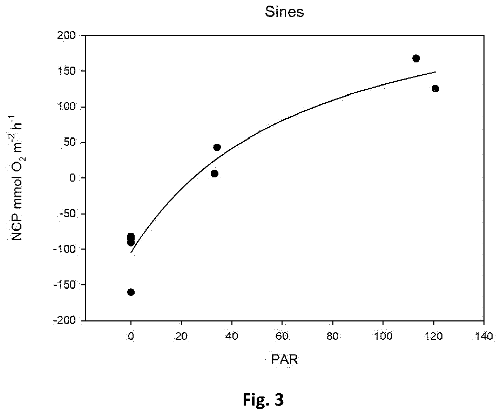

Data was collected with a prototype of a chamber system (according to Figure 1) which was deployed on varies rocky reefs along the coast of Portugal. The chamber was effectively sealed against the bottom and P-E (Photosynthesis-Irradiance) curves could be generated for algal communities of different heights (Figure 3).

In an embodiment, this technology can be used as a product to accurately measure community metabolic rates, i.e. primary production and respiration rates, in *situ.*

In another embodiment, the product could be attained by universities or research institutes as a basic system to regularly monitor functional trails of relevant communities.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

Where ranges are provided, the range limits are included. Furthermore, it should be understood that unless otherwise indicated or otherwise evident from the context and/or understanding of a technical expert, the values which are expressed as ranges may assume any specific value within the ranges indicated in different achievements of the invention, at one tenth of the lower limit of the interval, unless the context clearly indicates the contrary. It should also be understood that, unless otherwise indicated or otherwise evident from the context and/or understanding of a technical expert, values expressed as range may assume any sub-range within the given range, where the limits of the sub-range are expressed with the same degree of precision as the tenth of the unit of the lower limit of the range.

The following dependent claims further set out particular embodiments of the disclosure.

## Claims

1. Portable in-situ underwater metabolic chamber for measuring metabolic parameters of an underwater sample comprising:
a flexible incubation bag (4);
a top basal frame (1) and a bottom basal frame (2);
a sealer frame (3);
a flushing system (5) for renovating the sample inside the incubation bag;
and at least a sensor inside the flexible incubation bag for measuring the metabolic parameters, preferably a plurality of sensors;
wherein:
the flexible incubation bag comprises a closed top (7) and an open bottom (6);
the top basal frame (1) is contiguous to the bottom basal frame (2);
the sealer frame (3) is contiguous to the bottom basal frame (2);
the top basal frame (1), the bottom basal frame (2) and the sealer frame (3) surround the open bottom (6) of the flexible incubation bag (4).

2. Portable in-situ underwater metabolic chamber according to the previous claim wherein the sensor is a temperature sensor, a carbon dioxide concentration sensor, a pH sensor and/or an oxygen concentration sensor; preferably an oxygen concentration sensor.

3. Portable in-situ underwater metabolic chamber according to any of the previous claims wherein the flushing system (5) is connected to the flexible incubation bag (4); preferably to the bottom of the flexible incubation bag (4).

4. Portable in-situ underwater metabolic chamber according to any of the previous claims wherein the top frame comprises a rubber joint.

5. Portable in-situ underwater metabolic chamber according to any of the previous claims, wherein the length of the flexible incubation bag ranges from 50-200 cm; preferably 60-150 cm; more preferably 120 cm.

6. Portable in-situ underwater metabolic chamber according to any of the previous claims, wherein the diameter of the flexible incubation bag ranges from 40-350 cm; preferably 40-80 cm; more preferably 50 cm.

7. Portable in-situ underwater metabolic chamber according to any of the previous claims, wherein the flexible incubation bag (4) is transparent or translucid; preferably transparent.

8. Portable in-situ underwater metabolic chamber according to any of the previous claims wherein the sealer frame (3) further comprises a foam joint and a surrounding plastic material (to seal the chamber to the substrate); preferably the material of the foam joint of the sealer frame (3) is selected from a list consisting of: polyurethane, polyether, polyester, latex; preferably polyurethane.

9. Portable in-situ underwater metabolic chamber according to the previous claim wherein the material of the surrounding plastic material is selected from a list consisting of: high-density polyethylene, polyethylene terephthalate, polyvinyl chloride; preferably polyvinyl chloride.

10. Portable in-situ underwater metabolic chamber according to any of the previous claims, wherein the material of the flexible incubation bag (4) is selected from a list consisting of: low-density polyethylene, polypropylene; preferably polypropylene.

11. Portable in-situ underwater metabolic chamber according to any of the previous claims, wherein the top basal frame (1) and/or the bottom basal frame (2) has a circular shape.

12. Portable in-situ underwater metabolic chamber according to any of the previous claims, wherein the internal diameter of the top basal frame (1) and/or the internal diameter of the bottom basal frame (2) ranges from 40 - 80 cm; preferably 50 cm.

13. Portable in-situ underwater metabolic chamber according to any of the previous claims, wherein the external diameter of the top basal frames (1) and/or the external diameter of the bottom basal frame (2) ranges from 60 - 100 cm; preferably 70 cm.

14. Portable in-situ underwater metabolic chamber according to any of the previous claims, wherein
the diameter of the flexible incubation bag (4) is 50 cm;
the material of the top basal frame (1) and/or the material of the bottom basal frame (2) is polyvinyl chloride and comprises a circular shape;
the internal diameter of the top basal frame (1) and/or the internal diameter of the bottom basal frame (2) is 50 cm; and
the external diameter of the top basal frame (1) and/or the external diameter of the bottom basal frame (2) is 70 cm.

15. Use of the portable in-situ underwater metabolic chamber according to any of the previous claims as a metabolic chamber for analyzing *in situ* a substrate in pelagic and benthic habitats.
